# EUROPEAN PATENT APPLICATION

(11) **EP 4 105 176 A1**
(43) Date of publication of application: **21.12.2022**
(21) Application number: 21753701.8
(22) Date of filing: 09.02.2021
(51) Int. Cl.: C01B 32/182, C01B 32/194, C12Q 1/00

(54) **COVALENT FUNCTIONALISATION OF GRAPHENE**

(30) Priority: 11.02.2020 ES 202030111
(71) Applicant: Universidad Rey Juan Carlos, 28933 Mostoles (Madrid) (ES)
(72) Inventor: ÁLVAREZ CASTILLO, Ángel Luis, 28231 Madrid (ES); QUESADA SÁNCHEZ, Sergio Javier, 23006 Jaén (ES); BORRÁS RODRIGO, Fernando, 28342 Madrid (ES); COYA PÁRRAGA, Carmen, 28050 Madrid (ES)
(74) Representative: Manuel Illescas y Asociados, S.L.
(86) International application number: PCT/ES2021/070088
(87) International publication number: WO 2021/160911

(57) **Abstract**

The present invention relates to a process for obtaining covalently modified graphene. The invention also describes covalently modified graphene obtained by this process and use thereof as an anchoring surface for bioreceptors in biosensor devices. Lastly, the invention describes a biosensor device comprising covalently modified graphene obtainable according to the process of the invention.

## Description

### TECHNICAL FIELD OF THE INVENTION

The present invention relates to a process for obtaining covalently modified graphene. Likewise, the present invention relates to the covalently modified graphene obtainable by this process and use thereof as an anchoring surface for bioreceptors in biosensor devices. Lastly, the present invention describes to a biosensor device comprising covalently modified graphene obtainable according to the process of the invention.

### STATE OF THE ART

Currently, graphene has been functionalised, both covalently and non-covalently, (Plutnar et al, J. Mater. Chem. C., 2018, 6, 6082-6101) with a variety of molecules, even reaching the anchorage of antibodies, but in most cases it has been reactions in liquid phase (in dissolution), where the graphene used is in the form of flakes in suspension or forming part of a submerged electrode. This has resulted, in the last 5 years, in a large number of publications (Georgakilas et al., Chem. Rev. 2016, 116, 5464-5519). There are also publications wherein said graphene functionalisation is carried out outside a dissolution cuvette, in solid phase on previously deposited layers of graphene, or graphene oxide. However, these methods do not allow a positional control of the modifications made on the surface of graphene, or graphene oxide, without differentiating regions or separate areas within said layers (Morales-Narvaez et al., Adv. Mater., 2018, 1805043; Sandoval et al., Chem. Commun., 2019, 55, 12196-12199). The oxidation lithography technique, known as local anodic oxidation (LAO), or oxidation scanning probe lithography (o-SPL), does allow positional control. However, this technique has so far only been applied (1)on a nanometric scale by adapting microscopes that work at this scale, being limited to very small and impractical areas, of the order of nm (oxidation lithography, R. Garcia et al., Carbon 2018, 129, 281-285; Alvarez et al., 2017, Spanish Conference on Electron Devices (CDE), p. 1-4, doi:10.1109/CDE.2017.7905231); and (2) only for the purpose of oxidation, not extended to other types of functionalisations or chemical reactions.

Very recently, a way to functionalise graphene in differentiated areas has been published, through a process with light irradiation (Valenta et al., Agew. Chem. Int. Ed, 2019, 58, 1324-1328). In this method, the reaction is favoured only in those areas wherein a mask is used, being able to locate the modified region in various positions of the sample surface. However, this process is very limited to certain specific reactions susceptible of being carried out by light irradiation in the blue or UV region, such as those known as Mitsunobu reactions.

On the other hand, WO 2018098286 A1 discloses biomolecular sensors, which make use of graphene surfaces (and defective graphene). However, the bonding of the sensor molecules (in this case polymerase enzymes) to graphene, as described in said document requires, for positional control, the use in the sensor of masks, and only describes the modification of graphene, prior to its use in the sensor, without any positional control (dispersion of nanoparticles, or chemical etching) or with the use of masks to be able to carry out such control.

WO 2018013586 A1 does not disclose the covalent bonding of sensor molecules to graphene, but uses a functionalisation layer deposited on the layer of graphene that is the one that is covalently bonded to the molecules or functional groups that bond to the biomolecules of interest of the sensor. However, the graphene disclosed in said document is not covalently functionalised.

US2019/0079068 A1 and WO2016054550 disclose sensor devices that can use as a sensor element a carbon conductive material, such as carbon nanotubes, fullerenes or graphene. Furthermore, said documents disclose the use of protein- or antibody-functionalised graphene, wherein said graphene is bound to the proteins or antibodies directly or via a linker, and indicates that said bonding can be covalent. However, in no case does said document disclose how said functionalisation is performed, or if there is any, or how to perform a positional control thereof.

Thus, a method of functionalising graphene having the characteristics of positional control and versatility as to the nature of the reagent used has not yet been disclosed, wherein such a method would allow providing a physical substrate whereon to anchor different molecules or functional groups which, for example, can be biomarkers or bioreceptors (such as antibodies), on a single miniaturised platform, making said assays much more compact in size, and providing a system that allows the detection of analytes in a small space, compatible for developing lab-on-chip systems, i.e. systems that integrate and automate several laboratory techniques in a chip-sized device with a maximum size of a few square centimetres.

### BRIEF DESCRIPTION OF THE INVENTION

The present invention relates to a process for obtaining a covalently modified graphene, comprising:
i. applying an electric voltage of at least 10 volts generating an electric field in an area of the graphene surface in the presence of water and a substance which, in water, or in water and in the presence of the electric field generated by said voltage, gives rise to one or more ions and/or to one or more radicals;
ii. optionally washing the graphene and,

repeating step (i) and (ii) successively in another area of the graphene surface to be modified, different from the area or areas wherein said steps (ii) and (ii) have already been performed, with the same or another different substance that, in water, or in water and in the presence of the electric field generated by said voltage, gives rise to one or more ions and/or to one or more radicals;;
and wherein the substance which, in water, or in water and in the presence of an electric field, gives rise to one or more ions and/or to one or more radicals, is independently selected from the group consisting of:
   - a compound which, in water, or in water and in the presence of an electric field gives rise to a radical selected from the group consisting of: superoxide radical, alkoxyl radical, aroxyl radical, peroxyl radical, aminoxyl radical, nitrogen monoxide radical, nitrogen dioxide radical, nitrosonium cation, nitrene radical, halogen radical, alkyl radical, aryl radical, carbene radical and cetyl radical; or a compound comprising one or more functional groups which, in water, or in water and in the presence of an electric field, give rise to said radical;
   - a compound which, in water, or in water and in the presence of an electric field, gives rise to a cation, wherein said cation is independently selected from the group consisting of: a metal cation, an ammonium cation, a nitronium cation, a carbocation, a diazonium cation and a guanidine cation; or a compound comprising one or more functional groups which, in water, or in water and in the presence of an electric field, give rise to said cation; and
   - a compound which, in water, or in water and in the presence of an electric field, gives rise to an anion, wherein said anion is independently selected from the group consisting of: peroxide, alkoxide, phenolate, carboxylate, azide, carbonate, bicarbonate, borate, bromate, iodate, cyanate, isocyanate, cyanide, nitrate, nitrite, halide, sulphate, bisulphate, thiosulphate, sulphite, bisulphite, sulphide, bisulphide, disulphide, phosphate, phosphite, acid phosphate, diacid phosphate and silicate; or a compound which comprises one or more functional groups which, in water, or in water and in the presence of an electric field, give rise to said anion.

The present invention also relates to a process for obtaining covalently modified graphene, comprising:
i. applying an electric voltage of at least 10 volts generating an electric field in an area of the graphene surface in the presence of water and a substance which, in water, or in water and in the presence of the electric field generated by said voltage, gives rise to one or more ions and/or to one or more radicals;
ii. optionally washing the graphene and,

repeating step (i) and (ii) successively in another area of the graphene surface to be modified, different from the area or areas wherein said steps (ii) and (ii) have already been performed, with the same or another different substance which, in water, or in water and in the presence of the electric field generated by said voltage, gives rise to one or more ions and/or to one or more radicals;
and wherein the substance which, in water, or in water and in the presence of an electric field, gives rise to one or more ions and/or to one or more radicals, is independently selected from the group consisting of:
   - a compound which, in water, or in water and in the presence of an electric field, gives rise to a radical selected from the group consisting of: alkoxyl radical, aroxyl radical, nitrosonium cation, nitrogen radical, halogen radical, aryl radical, and carbon radical; or a compound that comprises one or more functional groups which, in water, or in water and in the presence of an electric field, give rise to said radical;
   - a compound which, in water, or in water and in the presence of an electric field, gives rise to one or more cations independently selected from the group consisting of: Li⁺, Na⁺, K⁺, Mg²⁺, Ca²⁺, Cu²⁺ ammonium, diazonium and carbocation; or a compound comprising one or more functional groups that in water, or in water and in the presence of an electric field, give rise to said cation; and
   - a compound which, in water, or in water and in the presence of an electric field, gives rise to one or more anions independently selected from the group consisting of: chloride, iodide, bicarbonate, alkoxide, phenolate and carboxylate; a compound which comprises one or more functional groups which in water, or in water and in the presence of an electric field, give rise to said anion.

Likewise, the present invention relates to the covalently modified graphene, obtainable according to the process of the present invention.

Another aspect of the present invention relates to the use of the covalently modified graphene, obtainable in accordance with the process of the present invention, in an analyte detector device.

Finally, the present invention relates to an analyte detecting device comprising covalently modified graphene, obtainable according to the process of the present invention.

### BRIEF DESCRIPTION OF THE FIGURES

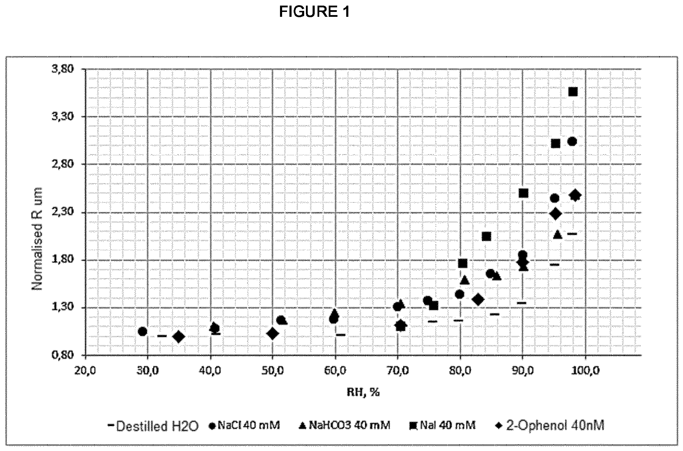

**FIG. 1****.** Comparison of relative humidity versus normalised area radius of graphene surface covalently modified with NaCl, NaHCO₃, Nal and 2-chlorophenol (all at 40 mM) versus a control with distilled water.

### DETAILED DESCRIPTION

The present invention relates to a process for obtaining covalently modified graphene, comprising: (i) applying an electric voltage of at least 10 volts to generate an electric field in an area of the graphene surface in the presence of a solvent and a substance which, in said solvent, or in said solvent and in the presence of the electric field generated by said voltage, gives rise to one or more ions and/or to one or more radicals.

The term "solvent" refers to a chemical compound that, when mixed with another compound or substance, forms a homogeneous mixture, constituting a single phase, i.e. it forms a mixture wherein the physical properties remain homogeneous, understanding by "homogeneous" that whereby all the intensive properties (those that do not depend on the amount of substance) are constant. In general, the amount of solvent in the mixture is greater than that of the other substance, which is generally called a solute and is, therefore, a substance other than the solvent. Preferably said solvent is water.

The term "substance which, in a solvent, or in a solvent and in the presence of an electric field, gives rise to one or more ions and/or to one or more radicals" refers to a substance other than the solvent which, in contact with said solvent and, optionally in the presence of an electric field (generated by the application of a voltage), generates at least one ion and/or at least one radical. Said term thus comprises both ionic substances (i.e. substances comprising ionic bonds) which, being dissolved in said solvent, release the cations and anions that form it and, also comprises substances with covalent bonds, which are in suspension, or vaporised in said solvent and which, as they are in contact with said solvent, or as they are in contact with said solvent and in the presence of an electric field, generate the formation of at least one ion and/or the formation of at least one radical, and. In other words, in some cases, the substance used gives rise to ions or radicals simply by being dissolved in the solvent used, while, in other cases, the presence of an electric field will be necessary for said substance dissolved in said solvent to give rise to ions or radicals.

In a preferred embodiment of the invention, once step (i) has been performed, said step (i) is repeated, successively, in another area of the graphene surface to be modified, different from the area or areas wherein it has already been performed, in the presence of the same solvent or another different solvent and, with the same substance, or another different substance that, in the solvent used, or in the solvent used and in the presence of the electric field generated by said voltage, gives rise to one or more ions and/or to one or more radicals.

In this way, the present invention allows the covalent modification of graphene , at one or more points, and with one or more types of functional groups, since said method allows the covalent modification of graphene with a wide variety of substances that are dissolved, or in suspension, in the solvent used, and in particular in water (i.e. when the solvent is water), and furthermore, allows the modification of graphene in one or more areas, said one or more areas comprising the same or different functional group. Unlike the graphene modifications disclosed in the state of the art, the present process makes it possible to avoid the use of templates, which require the use of additional steps. Furthermore, the current method does not require the use of solutions and substances that potentially damage the graphene layer, since the modification is located spatially. In this way, the process of the invention allows obtaining covalently modified graphene with different functional groups in different specific areas of its surface, wherein both the introduced groups, as well as the modified areas, can be selected by the user of the process.

In one embodiment, the process comprises:
i. applying an electric voltage of at least 10 volts to generate an electric field in an area of the graphene surface in the presence of a solvent and a substance which, in said solvent, or in said solvent and in the presence of the electric field generated by said voltage, gives rise to one or more ions and/or one or more radicals; and
ii. washing the graphene.

In one embodiment of the invention, once step (ii) has been performed, step (i) and, optionally, step (ii) are repeated, successively, in another area of the graphene surface to be modified, different from the area or areas wherein it has already been performed, in the presence of the same solvent or another different solvent and, with the same or another different substance that, in the solvent used, or in the solvent used and in the presence of the electric field generated by said voltage, gives rise to one or more ions and/or one or more radicals.

Thus, the process of the present invention comprises:
i. applying a voltage of at least 10 volts generating an electric field in an area of the graphene surface in the presence of a solvent and a substance which, in said solvent, or in said solvent and in the presence of the electric field generated by said voltage, gives rise to one or more ions and/or to one or more radicals;
ii. washing the graphene and/or, optionally, repeating the previous step successively in another area of the graphene surface to be modified, different from the area or areas wherein the previous step (i) has already been performed, in the presence of the same solvent or another different solvent and, with the same substance, or another different substance that, in the solvent used, or in the solvent used and in the presence of the electric field generated by said voltage, also gives rise to one or more ions and/or to one or more radicals.

Thus, the process of the invention comprises covalent modification in an area of the graphene, according to step (i), and wherein said step (i) can be repeated, comprising, between each step (i), a step (ii) wherein the graphene is washed, prior to carrying out step (i) again, or not.

In other words, the process of the invention comprises a step (i), optionally a step (ii), wherein step (i), and optionally step (ii), is repeated successively in several areas of the graphene surface, in the presence of the same solvent or different solvents, and of the same substance or a different substance, each time step (i) is carried out.

In a preferred embodiment of the invention, the solvent is water and the invention relates to a process of obtaining covalently modified graphene, comprising:
i. applying an electric voltage of at least 10 volts to generate an electric field in an area of the graphene surface, in the presence of water comprising a substance which, in water, or in water and in the presence of the electric field generated by said voltage, gives rise to one or more ions and/or to one or more radicals;
ii. optionally, washing the graphene.

In one embodiment of the invention, step (i), and optionally step (ii), are repeated, successively, in another area(s) of the graphene surface to be modified, different from the area are areas wherein it has already been performed, with the same substance or another different substance which, in water, or in water and in the presence of the electric field generated by said voltage, also gives rise to one or more ions and/or to one or more radicals.

In other words, in a more preferred embodiment of the invention, the process further comprises repeating step (i) and (ii) successively in another area of the graphene surface to be modified, different from the area or areas wherein said steps (i) and (ii) have already been performed, with the same or another different substance that, in water, or in water and in the presence of the electric field generated by said voltage, gives rise to one or more ions and/or to one or more radicals.

Thus, in a preferred embodiment of the invention, the covalent modification can preferably be carried out in the presence of water vapour, or of a fine liquid water atomisation comprising a substance which, in water, or in water and in the presence of an electric field, gives rise to at least one ion and/or at least one radical. Thus, the graphene surface is in contact with an atmosphere, called the working atmosphere, rich in water, which contains said substance.

For the purposes of the present invention, the term "comprises" indicates that it includes a group of features, but does not exclude the presence of other features, provided that the presence of the other features does not render the claim impracticable. In addition, the terms "consists of", "contains", "includes", "has", "encompasses" and synonyms of such terms are to be construed in the same manner as the term "comprises".

Additionally, for the purposes of the present invention, the term "comprises" may be replaced by any of the terms "consists of", or "substantially consists of". Thus, where the term "comprises" refers to a group of technical features A, B and C, it should be construed as including additionally other technical features in addition to technical features A, B and C, provided that the presence of the other features does not render the invention impracticable, but may also be construed as comprising only such features A, B and C or substantially such features A, B and C, and therefore the term "comprises" referring to a group comprising features A, B and C is to be construed as including a group consisting of features A, B and C, or consisting substantially of features A, B and C.

Thus, the solvent, optionally with the effect of the electric field generated by applying a voltage, produces the formation of ions (for example, the generation of a Cl⁻ anion and of the Na⁺ cation, when the NaCI salt is in the presence of water as a solvent or, for example, the formation of the R-NH ₃⁺ ammonium cation of an R-NH ₂ amine, by the bonding of an H⁺ proton to said amine in the presence of water), or of radicals, when a break occurs of a bond that generates species with unpaired electrons.

For the purposes of the present invention, the term "radical" refers to a chemical species that possesses one or more unpaired electrons. Examples of radicals, for the purposes of the present invention, include, but are not limited to: superoxide radical (O₂^{·-}), oxygen diradical (O₂^{··}), hydroxyl radical (HO·), alkoxyl radical (RO·), aroxyl radical (ArO ·), peroxyl radical (ROO ·), aminoxyl radical (R-N-O .) among which the TEMPO reagent (tetramethylpyridyl-N-oxyl) and its derivatives, nitrogen monoxide radical (NO·), nitrogen dioxide radical (NO₂·), nitrosonium cation (NO₂^{·+}), nitrogen radical (R-N·), halogen radical (X·), alkyl radical (R·), aryl radical (Ar·), carbon radical (R··), etc. can be cited; wherein R is an alkyl group or H, X is a halogen atom and Ar is an aryl group.

On the other hand, for the purposes of the present invention, the term "ion" refers to an electrically charged atom or group of atoms.

The process of the invention is carried out in the presence of an electric field generated by the application of a voltage. In some cases, said electric field is further necessary for the formation of ions and/or radicals of the substance used (e.g. for the formation of carbocations, or other types of ions or radicals that require greater energy for their formation in the presence of said solvent).

The covalent modification of the graphene occurs, as a result of the process of the invention, by forming new bonds between one or more graphene carbon atoms and one of the atoms of the ion, or of the radical, generated by said substance, forming functional groups covalently bonded to carbon atoms of the graphene, wherein the covalent bond is produced between one of the carbon atoms of the graphene and an atom of the ion, or of the radical of the substance used.

In a more preferred embodiment, the electric voltage of step (ii) is applied by a tip electrode connected to one of the poles of an electric voltage generator. For the purposes of the present invention, the term "electric voltage generator" is equivalent to a voltage source. In other words, the voltage is "applied" externally by means of a voltage generator (or voltage source), between two poles or electrodes, generating an electric field whose force lines go from one pole to another. When one of the electrodes ends at the tip, the electric field generated is concentrated on said tip.

For the purposes of the present invention, a tip electrode is an electrode whose end has a diameter of less than 30 microns.

In an even more preferred embodiment, the electric field is applied by a device comprising: a tip electrode connected to a first pole of an electric voltage generator, while a second pole of said generator is connected to the graphene; a head configured to hold the tip electrode; a dielectric separator housing said head and which is configured to electrically isolate the electrode from the rest of the device; a positioner which is fastened to the electric separator and which is configured to move the head in the three dimensions of the space; and a damping system configured to allow vertical movement of the head with a predetermined pressure and to damp the displacements resulting from the irregularities of the surface wherethrough the head is moved; wherein in step (ii) the electric field is generated with the tip electrode of said device; and a container with solvent configured to generate a dispersion of said solvent. In particular, said device is called a functionalisation lithography device.

In one embodiment of the invention, the electric field is generated by an electrode, in particular a tip electrode, with negative electrical potential with with respect to graphene.

In another embodiment of the invention, the electric field is generated by an electrode, in particular a tip electrode, with positive electrical potential with with respect to graphene.

In another embodiment of the invention, the electric field is generated by an electrode, in particular a tip electrode, with negative or positive electrical potential with with respect to graphene

For example, it can be theoretically explained that, when the electric field is generated by an electrode with negative potential with with respect to graphene (cathode), the graphene acts as a positive electrode (anode) and attracts the negative charges of the species (ions and/or radicals) generated by the electric field and binds to an atom of said species rich in negative charge, by means of a nucleophilic addition reaction. On the other hand, also by way of example, when the electric field is generated by an electrode with positive potential with with respect to graphene, the electron cloud n of the graphene attracts the positive charges of the species (ions and/or radicals) generated and binds to an atom of said species rich in positive charge by an electrophilic addition reaction.

Graphene is a single, two-dimensional layer of bonded carbon atoms forming hexagons by carbon-carbon bonds that exhibit sp² hybridisation and forming a n cloud of electrons. Due to its structure, graphene behaves as a semi-metal with a very high load mobility and has unique properties, among which hardness, elasticity, high conductivity with a very reduced resistance but a chemical structure, in principle, very stable (or not very reactive) to gases. This makes graphene a very interesting material in many technical applications. Preferably, the graphene may be single-layer graphene or few-layer graphene with 2 to 10 monolayers of graphene. For the purposes of the present invention, in the event that the graphene to be covalently modified in the process of the invention consists of more than one graphene monolayer, the term "a graphene surface" refers to any of the graphene monolayers. Although graphene is a two-dimensional material, in some cases, the material used comprises more than one layer of graphene. Thus, for the purposes of the present invention, when the graphene is formed by more than one layer, the covalent modification can occur in any of the graphene layers. The formation of a covalent bond between a carbon atom and a functional group involves the transition to a sp³ hybridisation of the reactive carbon atom. This results in materials with different properties and reactivity than the initial graphene.

In one embodiment the, the covalent modification is a monotopic modification (i.e. occurring only on one of the two faces of the graphene surface. In another embodiment, the covalent modification is a ditopic modification (i.e. occurs on either side of the graphene surface).

For the purposes of the present invention, the solvent comprising the substance which, in said solvent, or in said solvent and in the presence of an electric field, gives rise to one or more ions and/or to one or more radicals, is in contact with the graphene surface in the form of "atomisation" or "vaporisation" or "dispersion", without the use of a heat source being required, in such a way that an atmosphere is generated with said solvent and substance in contact with the graphene surface. In this way, the terms "atomisation", "vaporisation" or "dispersion" are used interchangeably, for the purposes of the present invention, and indicate the generation of a homogeneous phase, in terms of its physical properties, containing said solvent and said substance, without the need for the use of a heat source.

Thus, the process of the invention allows a positional control of the covalent anchoring of functional groups and, since it is an electrical process, based on the application of a localised electric voltage, it allows working with a wide variety of substances that generate ions or radicals when in contact with said solvent, or that generate said ions or radicals when, in addition, they are subjected to an electric field.

For the purposes of the present invention, the term "functional group" refers to an atom or set of atoms which, attached to the molecular structure of a compound of greater number of atoms, and arranged according to a given sequence of bonds, exhibit individualised and characteristic chemical properties. Thus, in the present invention said functional group refers to an atom or set of atoms of an ion or radical of a substance which, in a solvent, or in a solvent and in the presence of an electric field, gives rise to one or more ions and/or to one or more radicals, and which, during the process of the present invention, forms a covalent bond with a carbon atom of the graphene.

In a preferred embodiment of the process according to the present invention, the electric field is applied in a circular area of the graphene surface with a diameter of less than 15 microns.

In a preferred embodiment, the electric voltage is applied at a distance from the graphene surface of less than 10 nm, more preferably at a distance of less than 7 nm. In an even more preferred embodiment, the electric voltage is applied between a tip electrode and the graphene, keeping said electrode in contact with the surface of the graphene.

In one embodiment of the process according to the present invention, the electric voltage applied in the area of the graphene surface to be modified is between 10 and 100 volts; more preferably between 20 and 60 volts.

Thus, a preferred embodiment of the invention comprises (i) applying an electric voltage at a distance of less than 10 nm, more preferably at less than 7 nm, and even more preferably between a tip electrode and the graphene, said electrode being in contact with an area of the graphene surface, wherein said voltage is between 10 and 100 volts, more preferably between 20 and 60 volts, to generate an electric field in said area of the graphene surface in the presence of a solvent and a substance which, in said solvent, or in said solvent and in the presence of the electric field generated by said voltage, gives rise to one or more ions and/or one or more radicals.

In a preferred embodiment, the electric voltage is applied for a period of at least 1 millisecond (1 ms). In a more preferred embodiment, the electric voltage is generally applied for a period of between 1 ms and 30 seconds.

In a preferred embodiment of the invention, the solvent is water and step (i) of the process comprises:
i applying an electric voltage of between 10 and 100 volts, more preferably between 20 and 60 volts, between a tip electrode and graphene (acting as the other electrode), at a distance of less than 10 nm, more preferably less than 7 nm and even more preferably in contact with an area of the graphene surface, to generate an electric field in said area of the graphene surface, wherein said voltage is applied in the presence of water comprising a substance which, in water, or in water and in the presence of the electric field generated by said voltage, gives rise to one or more ions and/or to one or more radicals, and wherein the relative humidity in contact with the graphene surface to be modified is at least 40%, more preferably of at least 50% and even more preferably of between 60% and 95%.

For the purposes of the present invention, the term "atmosphere" or "working atmosphere" refers to the solvent phase in direct contact with the surface to be modified in accordance with the process of the present invention. The relative humidity values when the water is the solvent used, according to the present document, are expressed in %.

Thus, the application of said electric voltage generates an electric field in an area of the graphene surface, in the presence of a solvent comprising a substance which, in said solvent, or in said solvent and in the presence of an electric field, gives rise to one or more ions and/or to one or more radicals, generates the covalent modification of the graphene by forming one or more covalent bonds between carbon atoms of the graphene and one or more atoms of said substance or said solvent, in an area which preferably has a diameter of about 1 to 12 times the size of the area in which the electric voltage has been generated. Thus, if, for example, an electric field has been generated in a circular area of 10 microns in diameter, the covalent modification will occur in an area with a diameter of 10 to 120 microns.

Even more preferably, the solvent is water and step (ii) comprises applying an electric voltage of between 20 and 60 volts, in contact with an area of the graphene surface, in the presence of water comprising a substance which, in water, or in water and in the presence of an electric field gives rise to at least one ion and/or at least one radical, and wherein the relative humidity in contact with the graphene surface to be modified is at least 50% and more preferably between 60% and 99%.

In another embodiment of the invention, the solvent is a polar solvent. Preferably, a protic polar solvent. More preferably, the solvent is water, a carboxylic acid, an alcohol, an amine, or a hydrogen halide. Even more preferably, the solvent is water.

In this way, with the process of the invention, graphene can be functionalised, or covalently modified, in large regions, of mm² or greater, with different chemical species, being able to covalently bond different groups, or the same, in different areas separated from each other from the surface of the graphene by generating an electric field located in said areas in the presence of said solvent, or of another different solvent that contains the same substance, or with or different substances than in the solvent used, or in the solvent used and in the presence of an electric field, generate ions and/or radicals, and that form bonds with one or more carbon atoms of the graphene.

For example, when the solvent is water, the inorganic salts are ionised in water, a phase of fine liquid atomisation is generated wherein said salts are transported and respond to the generated electric fields. Thus, when applying an electric field located in the presence of said phase of fine liquid water atomisation, enriched with a salt, the electric field mediates in the bond of the ionic or radical species corresponding to graphene. To anchor two substances, one in each area, the process is carried out in a sequential manner: first, a first substance is used in a first area and the electric field is applied, resulting in the modification of the graphene with said first substance in said first area. Then, the graphene is washed and the process is repeated by working on another different area with a substance and solvent, the same or different from the first ones.

In one embodiment of the invention, the graphene is washed with DMF (dimethylformamide), DMSO (dimethyl sulphoxide), methanol, toluene or ethanol, preferably performing more than one wash with one or more solvents.

The process of the invention is not only possible with salts, in particular with inorganic salts, but can be carried out with any molecule, complex or substance that, in water, or other appropriate solvent, generates ions or radicals, requiring, or not, the presence of the electric field generated during the procedure, and without it being necessarily a molecule with bonds of an ionic nature.

Thus, in a preferred embodiment of the process according to the present invention, the substance which, in a solvent, or in a solvent and in the presence of an electric field, gives rise to one or more ions and/or to one or more radicals, is independently selected from the group consisting of:
- a compound which, in said solvent, or in said solvent and in the presence of an electric field gives rise to a radical selected from the group consisting of: superoxide radical, oxygen diradical, hydroxyl radical, alkoxyl radical, aroxyl radical, peroxyl radical, aminoxyl radical, nitrogen monoxide radical, nitrogen dioxide radical, nitrosonium cation, nitrene radical, halogen radical, alkyl radical, aryl radical, carbene radical and cetyl radical; or a compound comprising one or more functional groups which, in said solvent, or in said solvent and in the presence of an electric field, give rise to said radical,
- a compound which, in said solvent, or in said solvent and in the presence of an electric field gives rise to a cation selected independently from the group consisting of: a metal cation, an ammonium cation, a nitronium cation, an iminium cation, an iodine cation, a carbocation, a diazonium cation, and a guanidine cation; or a compound that comprises one or more functional groups which, in said solvent, or in said solvent and in the presence of an electric field, give rise to said cation; and

- a compound which, in said solvent, or in said solvent and in the presence of an electric field, gives rise to an anion independently selected from the group consisting of: peroxide, hydroxide, alkoxide, phenolate, carboxylate, carbanion, azide, carbonate, bicarbonate, borate, bromate, iodate, cyanate, isocyanate, cyanide, nitrate, nitrite, halide, sulphate, bisulphate, thiosulphate, sulphite, bisulphite, sulphide, bisulphide, disulphide, phosphate, phosphite, acid phosphate, diacid phosphate and silicate; or a compound which comprises one or more functional groups which, in said solvent, or in said solvent and in the presence of an electric field, give rise to said anion.

In this way, when an electric field is generated, at a distance of the order of nm from the surface area of the graphene, in the presence of a solvent comprising said substance, one or more covalent bonds between one or more graphene carbon atoms and one of the atoms of said substance are produced, forming functional groups covalently bonded to carbon atoms of the graphene, wherein the covalent bond is produced between one of the carbon atoms of the graphene and an atom of the substance. Thus, in a non-limiting example, if the substance is an aryl diazonium salt (aryl-N ₂⁺ X⁻), it could generate in aqueous solution and in the presence of an electric field, N₂ and an aryl carbocation, or an aryl radical, which would react with a carbon of the graphene forming a bond of said carbon atom and a carbon atom of the aryl group, thus forming an aryl functional group in the graphene.

In a preferred embodiment of the invention, the substance giving rise in the solvent, or in the solvent and in the presence of an electric field, to one or more ions is independently selected from the group consisting of:
- a compound which, in said solvent, or in said solvent and in the presence of an electric field gives rise to a radical selected from the group consisting of: hydroxyl radical, alkoxyl radical, aroxyl radical, nitrosonium cation, nitrene radical, halogen radical, aryl radical and carbene radical; or a compound comprising one or more functional groups which, in said solvent, or in said solvent and in the presence of an electric field, give rise to said radical;
- a compound which, in said solvent, or in said solvent and in the presence of an electric field, gives rise to one or more cations independently selected from the group consisting of: Li⁺, Na⁺, K⁺, Mg²⁺, Ca²⁺, Cu²⁺ ammonium, diazonium and carbocation; or a compound comprising one or more functional groups which, in said solvent, or in said solvent and in the presence of an electric field, give rise to said cation; and
- a compound which, in said solvent, or in said solvent and in the presence of an electric field, gives rise to one or more anions independently selected from the group consisting of: chloride, iodide, bicarbonate, hydroxide, alkoxide, phenolate and carboxylate; a compound which comprises one or more functional groups which, in said solvent, or in said solvent and in the presence of an electric field, give rise to said anion.

Thus, graphene could react with a cation (e.g. carbocation of diazonium salts) or with an anion (e.g. I⁻ or HCO₃⁻) that could be generated by the substance used, depending on whether the graphene is more or less nucleophilic than the anion generated.

In a more preferred embodiment of the invention, the process of obtaining covalently modified graphene comprises:
i. applying an electric voltage of at least 10 volts generating an electric field in an area of the graphene surface in the presence of water and a substance which, in water, or in water and in the presence of the electric field generated by said voltage, gives rise to one or more ions and/or to one or more radicals;
ii. optionally washing the graphene and,

repeating step (i) and (ii) successively in another area of the graphene surface to be modified, different from the area or areas wherein said steps (ii) and (ii) have already been performed, with the same or another different substance which, in water, or in water and in the presence of the electric field generated by said voltage, gives rise to one or more ions and/or to one or more radicals;
and wherein the substance which, in water, or in water and in the presence of an electric field, gives rise to one or more ions and/or to one or more radicals, is independently selected from the group consisting of:
   - a compound which, in water, or in water and in the presence of an electric field gives rise to a radical selected from the group consisting of: superoxide radical, alkoxyl radical, aroxyl radical, peroxyl radical, aminoxyl radical, nitrogen monoxide radical, nitrogen dioxide radical, nitrosonium cation, nitrene radical, halogen radical, alkyl radical, aryl radical, carbene radical and cetyl radical; or a compound comprising one or more functional groups which, in water, or in water and in the presence of an electric field, give rise to said radical;
   - a compound which, in water, or in water and in the presence of an electric field, gives rise to a cation, wherein said cation is independently selected from the group consisting of: a metal cation, an ammonium cation, a nitronium cation, a carbocation, a diazonium cation and a guanidine cation; or a compound comprising one or more functional groups which, in water, or in water and in the presence of an electric field, give rise to said cation; and
   - a compound which, in water, or in water and in the presence of an electric field, gives rise to an anion, wherein said anion is independently selected from the group consisting of: peroxide, alkoxide, phenolate, carboxylate, azide, carbonate, bicarbonate, borate, bromate, iodate, cyanate, isocyanate, cyanide, nitrate, nitrite, halide, sulphate, bisulphate, thiosulphate, sulphite, bisulphite, sulphide, bisulphide, disulphide, phosphate, phosphite, acid phosphate, diacid phosphate and silicate; or a compound which comprises one or more functional groups which, in water, or in water and in the presence of an electric field, give rise to said anion.

In another additional preferred embodiment of the invention, the substance giving rise in water to one or more ions in the presence of an electric field is independently selected from the group consisting of NaCl, Nal, NaHCO₃, NaOH, CuCl₂, NH₃, 2-chlorophenol, variamine blue and the sodium salt of fluorescein.

In one embodiment of the invention, the electric field is generated by an electrode, in particular a tip electrode, with negative electrical potential with with respect to graphene.

In another embodiment of the invention, the electric field is generated by an electrode, in particular a tip electrode, with positive electrical potential with with respect to graphene.

Preferably, in the process according to the present invention, the graphene surface to be covalently modified is a monolayer.

More preferably, in the process according to the present invention, the graphene is obtained by chemical vapour deposition (CVD).

The highest quality graphene is obtained in the form of monolayers by chemical vapour deposition (CVD), which can be transferred, with procedures known in the state of the art, onto any substrate.

Although graphene is a two-dimensional material, in some cases, the material used comprises more than one layer of graphene. Thus, for the purposes of the present invention, when the graphene is formed by more than one layer, the covalent modification can occur in any of the graphene layers.

In a particular embodiment of the invention, the process is carried out with substances soluble in the solvent used and comprises, prior to step (i), the steps of:
a. dissolving a substance in a solvent; and
b. spreading the obtained solution on the graphene surface;
c. drying the graphene surface to form a film of said substance on the graphene surface,
so that, by placing the surface of the graphene in contact with a solvent equal to or different from the solvent of step (a), the substance deposited on the graphene surface is dissolved and, by applying an electric voltage of at least 10 volts in said area of the graphene surface according to step (i) in the presence of said solvent.

In one embodiment of the invention, the solvent used in step (a) and step (i) are the same. In another embodiment of the invention, the solvent used in step (a) and step (i) are different.

In one embodiment, the step (c) of drying is carried out using a heat source.

In a preferred embodiment of the invention, both the first solvent and the second solvent are water and the process comprises prior to step (i), the steps of:
a. dissolving a substance which, in water, or in water and in the presence of an electric field, gives rise to one or more ions; and
b. spreading the obtained solution on the graphene surface;
c. drying the graphene surface so that a film of said substance is formed on the graphene surface,
so that by placing the graphene surface in contact with water, the substance deposited on the graphene surface in the water dissolves, and by applying an electric voltage of at least 10 volts in said area of the graphene surface, according to step (i), an electric field is generated in said area of the graphene surface in the presence of water.

For the purposes of the present invention, the graphene covalent modification process in accordance with the present invention is also referred to as a graphene covalent "functionalisation lithography process". Thus, for the purposes of the present invention, the device generating the electric voltage in the graphene covalent modification process of the present invention may be referred to as a graphene functionalisation lithography device.

Preferably, prior to step (i) the tip electrode is at a distance of equal to or less than 10 nm from the area of the graphene surface to be modified. More preferably, the tip electrode is in contact with the area of the graphene surface to be modified.

In an embodiment of the invention, the electric field is generated before the tip of the electrode is at a distance equal to or less than 10 nm, and is maintained when it comes into contact with the area of the graphene surface to be modified.

Once step (i) is completed said procedure is repeated, optionally in another area or areas of the graphene surface. In a preferred embodiment, step (i) is repeated in an area adjacent to the first area to be modified and with the same substance, so that the covalent modifications obtained overlap.

In another preferred embodiment, step (i) is repeated in an area away from the first area to be modified and with a different substance, so that covalent modifications of different chemical nature are obtained in separate areas of the graphene surface.

In an even more preferred embodiment, the tip electrode is in contact with the graphene surface during generation of the electric field of step (i). In a more preferred embodiment of this most preferred embodiment, the tip electrode is in contact with the graphene surface during generation of the electric field of step (i) by placing pressure on the graphene surface to be covalently modified.

The electrode approaches the sample until a sufficiently close distance, of less than 10 nanometers, or making contact with the surface, so that the electric field reduces the energy barrier (activation energy) of formation of the covalent bond between the graphene and the ion or radical generated by the substance.

The present invention also relates to covalently modified graphene, obtainable according to the process described in the present invention.

In a preferred embodiment, the graphene to be modified, and the covalently modified graphene, obtainable according to the process of the present invention, are deposited on a copper layer, or a silicon carbide layer, silicon oxide, quartz, silicon, teflon, polyethylene terephthalate or on a nickel layer, or on a copper/nickel alloy, or on a platinum/copper alloy, or on a copper/nickel/zinc alloy, or on a mineral surface or on a polymeric surface.

Preferably, the covalently modified graphene, obtainable according to the present invention, is in the form of a graphene monolayer. More preferably in the form of a graphene monolayer obtained by chemical vapour deposition which is covalently modified, obtainable according to the process of the invention.

The process of the invention, therefore, achieves the modification of graphene covalently with different types of functional groups, controlling the position of said groups. In this regard, the covalently modified graphene, obtainable according to the process described in the present invention allows providing selective active sites for bonding to different molecules. This makes said graphene useful as an anchoring surface of bioreceptors in detector devices of one or more analytes, such as ELISA immunoassay devices, wherein the covalently modified graphene, obtainable according to the process of the invention, serves as an anchoring surface for receptor substances (e.g. antigens) that are capable of bonding to analytes to be determined. Furthermore, the control of the position and type of covalent modifications (different functional groups) allows the anchoring or immobilisation of different receptor substances, being able to carry out the detection of several analytes on a very small surface. Thus, miniaturisation of multiple analyte or multianalyte detection platforms, is possible more economically and in solid phase.

Thus, the present invention also relates to the use of covalently modified graphene, obtainable according to the process described herein, in an analyte or biosensor detector device.

Furthermore, the present invention relates to an in vitro method for detecting analytes in a sample of a human or animal, wherein said method comprises anchoring said analytes to bioreceptors anchored to covalently modified graphene, obtainable according to the process described herein.

For the purposes of the present invention, said device or biosensor is an analysis device that can detect a signal corresponding to a molecule present in an in vitro sample of a human or animal, generating a measurable signal from said sample with the use of a transducer. Said device or biosensor comprises a platform, in particular a surface whereon a bioreceptor molecule, or bioreceptor, is anchored that is capable of bonding to or recognising the analyte whose presence is to be measured, and whereon, preferably, either the bioreceptor or the analyte is attached to or comprises a physically, chemically, electrically, magnetically, optically (fluorescence or colorimetry for example) or thermally detectable molecule. Thus, the bioreceptor has to be able to recognise the analyte to be measured, such as an enzyme, an antibody, DNA, RNA, or whole cells.

The conductive properties of graphene allow its use in sensors based on fluorescence transfer, electrochemistry, electron transfer, cyclic voltammetry, differential pulse voltammetry, surface plasmon resonance, field effect transistors (FET), surface enhanced raman dispersion (SERS) and impedance spectrometry. Thus, one embodiment relates to the use of the covalently modified graphene, obtainable according to the process described herein in an analyte detector device based on fluorescence transfer or field effect transistors.

In one embodiment, the device is a biosensor based on fluorescence transfer, electrochemistry, electron transfer, cyclic voltammetry, differential pulse voltammetry, surface plasmon resonance, field effect transistors (FET) and electrochemical impedance spectrometry.

In another embodiment, the device is a graphene field effect transistor (FET) biosensor, or a graphene Forster resonance energy transfer (FRET) biosensor, or an electrochemical impedance spectrometry device, an LDI-MS device, or a cyclic voltammetry device, or a differential pulse voltammetry device, or a surface plasmon resonance device, or an ELISA type device.

Preferably, the covalently modified graphene forms part of the anchoring surface of analyte bioreceptors to be detected in the devices described above.

In a preferred embodiment, the bioreceptor anchoring surface is a covalently modified graphene surface, obtainable according to the process described in the present invention, deposited on a copper layer, or a silicon carbide layer, or on a nickel layer, or on a copper/nickel alloy, or on a platinum/copper alloy, or on a copper/nickel/zinc alloy, or on a mineral surface or on a polymeric surface. Preferably, said polymeric surface is polymethylmethacrylate. More preferably, said mineral surface is quartz.

Such bioreceptors, or bioreceptor molecules, may be, without limitation, any protein, peptide, nucleic acid, polysaccharide, nucleotide, nucleoside, sugar, antigen, aptamer, hormone or antibody that may be used for diagnostic methods of any type of analyte present in humans or animals.

In one embodiment, the bioreceptor or bioreceptor molecule is a peptide, a protein, a nucleic acid, a nucleotide, a nucleoside, an oligonucleotide, a sugar, or a polysaccharide. In another embodiment, the bioreceptor or bioreceptor molecule is a glycoprotein, more particularly an immunoglobulin. In another additional embodiment, the bioreceptor or bioreceptor molecule is a nucleic acid, an oligonucleotide, a polynucleotide, a single-stranded DNA, a double-stranded DNA, an aptamer or an RNA.

More preferably, the covalently modified graphene, obtainable according to the process described in the present invention, comprises functional groups whereby molecules with bioreceptor function can be attached to an analyte detecting device.

In addition, another aspect of the invention therefore relates to an analyte detector device comprising the covalently modified graphene, obtainable according to the process described in the present invention. Preferably, the covalently modified graphene forms part of a bioreceptor anchoring surface of the analytes to be detected. More preferably, the covalently modified graphene comprises one or more functional groups for anchoring to bioreceptors of the analytes to be detected.

Another embodiment of the invention relates to the use of graphene obtainable according to the process of the invention in a field effect transistor, in a graphene field effect transistor (FET) biosensor, or in a surface enhanced raman dispersion (SERS) biosensor, or in a graphene Forster resonance energy transfer (FRET) biosensor, or in an electrochemical detection biosensor.

### EXAMPLES

### Example 1: Functionalisation of graphene with various substances

The device used for the covalent modification of graphene is equipped with three electromechanical positioners that allow positional control in the three directions of the space, and a tip electrode with a spring constant of 300 N/m. For each determined value of relative humidity and voltage, the covalent modification of the graphene is carried out in contact mode (electrode in contact with the graphene surface), using an oscilloscope to detect the moment of electrical contact. The tip is compressed < 3 µm after said contact, to avoid damage to the graphene, and subsequently separated. It is then moved to an adjacent area, and the process is repeated, so that it is possible to control the position and size of the modification by overlapping of individual oxidised areas. The voltage remains constant throughout the process, at -25 V. The samples used are CVD graphene deposited on a quartz surface. Chemical reagents used in the manufactures are listed in **Table 1 (A, B and C):**

**Table 1A:**

| **0.5 mM NaCl** | | **20 mM NaCl** | | **40 mM NaCl** | | **60 mM NaCl** | |
|---|---|---|---|---|---|---|---|
| **RH** | **norm r, µm** | **RH** | **norm r, µm** | **RH** | **norm r, µm** | **RH** | **reff** |
| 27.6 | 1.000 | 27.6 | 1.000 | 29.3 | 1.000 | 29.3 | 1.000 |
| | | 39.5 | 1.018 | 40.8 | 1.075 | | |
| 49.7 | 1.097 | 50.5 | 1.106 | 51.4 | 1.156 | 50.2 | 1.261 |
| 60.1 | 1.172 | 59.5 | 1.134 | 59.8 | 1.163 | 61.5 | 1.420 |
| 69.7 | 1.195 | 70.2 | 1.305 | 70.1 | 1.297 | 69.8 | 1.619 |
| 74.9 | 1.215 | 75.5 | 1.431 | 74.9 | 1.359 | 75.5 | 1.685 |
| 79.8 | 1.276 | 81.0 | 1.404 | 80.0 | 1.429 | 80.1 | 1.816 |
| 85.0 | 1.324 | 84.5 | 1.397 | 85.0 | 1.649 | 85.7 | 1.941 |
| 90.4 | 1.312 | 89.8 | 1.564 | 90.1 | 1.844 | 90.0 | 2.462 |
| 95.0 | 1.820 | 95.0 | 1.744 | 95.2 | 2.438 | 95.4 | 2.694 |
| 98.4 | 1.938 | 98.1 | 2.202 | 98.0 | 3.034 | 98.1 | 3.406 |

**Table 1B:**

| **0.5 mM NaHCO₃** | | **20 mM NaHCO₃** | | **40 mM NaHCO₃** | | **60 mM NaHCO₃** | |
|---|---|---|---|---|---|---|---|
| **RH** | **norm r, µm** | **RH** | **norm r, µm** | **RH** | **norm r, µm** | **RH** | **norm r, µm** |
| 45.6 | 1.000 | 45.6 | 1.000 | 40.5 | 1.000 | 40.5 | 1.000 |
| 50.0 | 1.239 | 50.5 | 1.130 | 51.6 | 1.167 | 51.8 | 1.174 |
| 60.0 | 1.274 | 61.4 | 1.307 | 59.8 | 1.241 | 60.3 | 1.526 |
| | | 69.7 | 1.425 | 70.5 | 1.342 | 70.0 | 1.648 |
| 76.8 | 1.306 | 76.1 | 1.436 | 75.0 | 1.377 | 75.8 | 1.724 |
| 80.2 | 1.393 | 80.0 | 1.491 | 80.7 | 1.587 | 82.0 | 1.838 |
| 85.3 | 1.427 | 85.9 | 1.611 | 85.8 | 1.637 | 87.7 | 2.036 |
| 90.1 | 1.554 | 90.3 | 1.824 | 90.1 | 1.733 | 90.8 | 2.134 |
| 95.3 | 1.656 | 96.0 | 2.178 | 95.5 | 2.072 | 95.7 | 2.685 |
| 98.3 | 1.903 | 98.1 | 2.562 | 98.3 | 2.480 | 98.4 | 3.075 |

**Table 1C:**

| **0.5 mM Nal** | | **20mM Nal** | | **40mM Nal** | | **60 mM Nal** | |
|---|---|---|---|---|---|---|---|
| **RH** | **norm r, µm** | **RH** | **norm r, µm** | **RH** | **norm r, µm** | **RH** | **norm r, µm** |
| 26.0 | 1.000 | 26.0 | 1.000 | 26.0 | 1.000 | 26.0 | 1.000 |
| 50.2 | 1.308 | | | | | 49.7 | 1.272 |
| 61.3 | 1.401 | | | | | 59.0 | 1.421 |
| 70.0 | 1.531 | 69.5 | 1.067 | 70.5 | 1.104 | 70.7 | 1.482 |
| 75.1 | 1.552 | 75.9 | 1.289 | 75.8 | 1.317 | 75.3 | 1.578 |
| 80.4 | 1.577 | 79.8 | 1.509 | 80.4 | 1.765 | 79.5 | 1.687 |
| 86.0 | 1.631 | 85.0 | 1.869 | 84.2 | 2.045 | 85.0 | 1.928 |
| 90.0 | 1.775 | 89.4 | 2.213 | 90.1 | 2.500 | 91.0 | 2.404 |
| 95.1 | 1.999 | 95.5 | 2.441 | 95.3 | 3.022 | 95.5 | 3.187 |
| 98.1 | 2.078 | 98.3 | 2.555 | 98.0 | 3.561 | 98.0 | 3.611 |

**Table 1 (A, B and C):** Covalently modified graphene radius values (normalised with the tip electrode radius measured at the lowest relative humidity for each series) using different inorganic sodium salts.

An aqueous solution of each reagent was prepared at the corresponding concentration (expressed as mM concentrations for salts and except for NH₃ expressed as % v/v). The resulting solution or mixture was stirred for 10 min. at room temperature and placed in the atomisation or dispersion device. The atomising device containing the solution was then placed in the device comprising the electrode, which is enclosed in an insulating chamber. The atomisation equipment was connected, until the corresponding relative humidity value was reached. The relative humidity value was monitored with a hygrometer-thermometer. When the desired relative humidity (RH) was reached, the process of the invention was initiated, keeping the RH constant. Once the process was completed, the insulating chamber was opened and the sample was washed by consecutive immersion in ethanol, methanol and water for one minute, and allowed to dry by heating on a hotplate for 3h at a temperature between 40 - 70°C.

The expansion rate of the covalent modification of the graphene was measured as the radius of the spots or areas generated using said reagent at the corresponding concentration and humidity. The radius was normalised with the radius of the tip electrode used (in **Table 1, "norm r"** column), which was estimated as the spot generated at ambient RH, where there is no expansion of the covalent modification (first row of each group in **Table 1**). Each measurement was repeated with 10 spots and the results were averaged. The radii were measured using a differential contrast microscope (DIC).

**Figure 1** shows the comparison of the normalised radii using the sodium salts listed in **Table 1.** Three effects are observed:
(1) For the same reagent and concentration, the spot radius increases with RH. There is a first phase of slow growth (40 < RH < 80%), and a second phase of exponential growth (RH > 85%). This is because the water meniscus generated between the tip and the graphene is larger at higher RH.
(2) For the same reagent and RH, the covalent modification of the graphene is expanded with a higher concentration of compound (**Table 1**), being in any case greater than the radii with distilled water (**Table 2**).
(3) For the same value of RH and reagent concentration, the radius of the spot is greater the greater the nucleophilia of the chemical reagent used. In the case of the compounds of **Table 1,** nucleophilia (polarisability) grows in the order HCO₃ < Cl < I.

This nucleophilia is associated with the reactivity of the anion additive with respect to its covalent bonding, in the event that a mechanism equivalent to that of oxidation with distilled water is followed. The results (2) and (3) lead us to conclude that there is a phenomenon of covalent bonding to the graphene of the anions.

To confirm these results, experiments were carried out with new chemical reagents, such as ammonia or 2-chlorophenol, nucleophilic reagents that again had to expand the covalent modification with respect to distilled water, and others such as ethanol, less nucleophilic than water and which, therefore, should slow growth. The results confirm our conclusion. By way of example, some data selected in **Table 2** are collected.

**Table 2. Values of radii normalised with the radius of the tip for different humidity values using distilled water (white to estimate the speed increase), as well as other additives to confirm the results obtained in Table 1. In the case of ammonia, the radii appear to be somewhat lower than expected in view of the high concentration that was used. This may be due to their volatility, which causes some of the volume to be lost in the atomisation process.**

| **Distilled H₂O** | | **2-chlorophenol, 40 mM** | | **NH₃, 10% (V/V)** | |
|---|---|---|---|---|---|
| **RH** | **norm r**, **µm** | **RH** | **norm r, µm** | **RH** | **norm r, µm** |
| 32.5 | 1.0000 | 34.9 | 0.999 | 38 | 1.08152286 |
| 41.0 | 1.0221 | 50.0 | 1.032 | 50.9 | 1.22154485 |
| 50.0 | 1.0142 | | | 56 | 1.38641642 |
| 61.0 | 1.0058 | 70.5 | 1.115 | 70 | 1.52463555 |
| 71.0 | 1.1018 | | | 70.5 | 1.5436862 |
| 75.8 | 1.1451 | 82.8 | 1.385 | 75.3 | 1.65442348 |
| 79.8 | 1.1525 | 90.0 | 1.772 | 80.1 | 1.7727001 |
| 85.6 | 1.2219 | 95.2 | 2.281 | 84.5 | 1.77437272 |
| 89.9 | 1.3365 | 98.4 | 2.474 | 90.2 | 1.94661939 |
| 95.2 | 1.7413 | | | 94.4 | 2.07939856 |
| 98.0 | 2.0646 | | | 98.3 | 2.14594237 |

## Claims

1. Process for obtaining covalently modified graphene, comprising:
i. applying a voltage of at least 10 volts generating an electric field in an area of the graphene surface in the presence of water and a substance which, in water, or in water and in the presence of the electric field generated by said voltage, gives rise to one or more ions and/or to one or more radicals;
ii. optionally washing the graphene and,
repeating step (i) and (ii) successively in another area of the graphene surface to be modified, different from the area or areas wherein said steps (ii) and (ii) have already been performed, with the same or another different substance which, in water, or in water and in the presence of the electric field generated by said voltage, gives rise to one or more ions and/or to one or more radicals;
and wherein the substance which, in water, or in water and in the presence of an electric field, gives rise to one or more ions and/or to one or more radicals, is independently selected from the group consisting of:
- a compound which, in water, or in water and in the presence of an electric field gives rise to a radical selected from the group consisting of: superoxide radical, alkoxyl radical, aroxyl radical, peroxyl radical, aminoxyl radical, nitrogen monoxide radical, nitrogen dioxide radical, nitrosonium cation, nitrene radical, halogen radical, alkyl radical, aryl radical, carbene radical and cetyl radical; or a compound comprising one or more functional groups which, in water, or in water and in the presence of an electric field, give rise to said radical;
- a compound which, in water, or in water and in the presence of an electric field, gives rise to a cation, wherein said cation is independently selected from the group consisting of: a metal cation, an ammonium cation, a nitronium cation, a carbocation, a diazonium cation and a guanidine cation; or a compound comprising one or more functional groups which, in water, or in water and in the presence of an electric field, give rise to said cation; and
- a compound which, in water, or in water and in the presence of an electric field, gives rise to an anion, wherein said anion is independently selected from the group consisting of: peroxide, alkoxide, phenolate, carboxylate, azide, carbonate, bicarbonate, borate, bromate, iodate, cyanate, isocyanate, cyanide, nitrate, nitrite, halide, sulphate, bisulphate, thiosulphate, sulphite, bisulphite, sulphide, bisulphide, disulphide, phosphate, phosphite, acid phosphate, diacid phosphate and silicate; or a compound which comprises one or more functional groups which, in water, or in water and in the presence of an electric field, give rise to said anion.

2. Process according to claim 1, wherein the electric voltage applied in the area of the graphene surface to be covalently modified is between 20 and 60 volts.

3. Process according to any of claims 1 or 2, wherein step (i) comprises applying an electric voltage of between 20 and 60 volts, between a tip electrode and the graphene, at a distance of less than 7 nm from an area of the graphene surface, to generate an electric field in said area of the graphene surface, wherein said voltage is applied in the presence of water comprising a substance which, in water, or in water and in the presence of the electric field generated by said voltage, gives rise to one or more ions and/or to one or more radicals, and wherein the relative humidity in contact with the graphene surface to be modified is at least 50%.

4. Process according to claim 3, wherein the electric field is generated by a tip electrode with negative electric potential with with respect to graphene or with positive electric potential with with respect to graphene.

5. Process according to any of claims 1 to 4, wherein the graphene surface to be covalently modified is of a graphene monolayer.

6. Process according to any of claims 1 to 5, wherein the graphene surface to be modified is obtained by chemical vapour deposition.

7. Process according to any of claims 1 to 6, wherein the electric field is generated in an area of the surface of the graphene to be modified of a diameter of less than 30 microns.

8. Process according to any of claims 1 to 7, wherein the electric voltage is applied prior to contact with the graphene surface area to be modified, and maintained during said contact.

9. Process according to any of claims 1 to 8, wherein the electric voltage is applied for a period of at least 1 millisecond.

10. Process according to any of claims 1 to 9, comprising prior to step (i), the steps of:
a. dissolving a substance which, in water, or in water and in the presence of an electric field, gives rise to one or more ions; and
b. spreading the obtained solution on the graphene surface;
c. drying the graphene surface so that a film of said substance is formed on the graphene surface,
so that by placing the graphene surface in contact with water, the substance deposited on the graphene surface in the water dissolves, and by applying an electric voltage of at least 10 volts in said area of the graphene surface, according to step (i), an electric field is generated in said area of the graphene surface in the presence of water.

11. Process according to any of claims 1 to 10, wherein the substance which, in water, or in water and in the presence of an electric field, gives rise to one or more ions and/or to one or more radicals, is independently selected from the group consisting of:
- a compound which, in water, or in water and in the presence of an electric field, gives rise to a radical selected from the group consisting of: alkoxyl radical, aroxyl radical, nitrosonium cation, nitrene radical, halogen radical, aryl radical and carbene radical; or a compound comprising one or more functional groups which, in said solvent, or in said solvent and in the presence of an electric field, give rise to said radical;
- a compound which, in water, or in water and in the presence of an electric field, gives rise to a cation, wherein said cation is independently selected from the group consisting of: Li⁺, Na⁺, K⁺, Mg²⁺, Ca²⁺, Cu²⁺, an ammonium cation, a diazonium cation and a carbocation; or a compound comprising one or more functional groups which, in water, or in water and in the presence of an electric field, give rise to said cation; and
- a compound which, in said solvent, or in said solvent and in the presence of an electric field, gives rise to an anion, wherein said anion is independently selected from the group consisting of: chloride, iodide, bicarbonate, alkoxide, phenolate and carboxylate; or a compound comprising one or more functional groups which, in water, or in water and in the presence of an electric field, give rise to said anion.

12. Process according to any of claims 1 to 11, wherein the relative humidity of the dispersion in contact with the graphene surface area to be modified is between 60 and 99%.

13. Process according to any of claims 1 to 12, wherein the electric field is applied by a device comprising: a tip electrode connected to a first pole of an electric voltage generator, a second pole of said generator connected to the graphene; a head configured to hold the tip electrode; a dielectric separator, housing said head, and configured to electrically isolate the electrode from the rest of the device; a positioner which is fastened to the electric separator and which is configured to move the head in the three dimensions of the space; and a damping system configured to allow vertical movement of the head with a predetermined pressure and to damp the displacements resulting from irregularities of the surface wherethrough the head is moved; wherein in step (ii) the electric field is generated with the tip electrode of said device; and a container with solvent configured to generate a dispersion of said solvent.

14. Covalently modified graphene obtained according to the process of any of claims 1 to 13.

15. Use of the covalently modified graphene of claim 14, in an analyte detector device.

16. Use according to claim 15, wherein the covalently modified graphene forms part of the anchoring surface of bioreceptor molecules of analytes to be detected.

17. Use according to any of claims 15 or 16, wherein the covalently modified graphene comprises functional groups for bonding to bioreceptor molecules in said analyte detecting device.

18. An analyte detector device comprising the covalently modified graphene according to claim 14.

19. An analyte detector device according to claim 18, wherein the covalently modified graphene forms part of an anchoring surface of bioreceptor molecules of the analytes to be detected.

20. An analyte detector device according to any of claims 18 or 19, wherein the covalently modified graphene comprises one or more functional groups for anchoring to bioreceptor molecules of the analytes to be detected.
